(19) Europäisches Patentamt
European Patent Office
Office européen des brevets

(11) **EP 4 094 684 A1**

(12) # EUROPEAN PATENT APPLICATION

(43) Date of publication:
**30.11.2022 Bulletin 2022/48**

(21) Application number: **21305716.9**

(22) Date of filing: **28.05.2021**

(51) International Patent Classification (IPC):
**A61B 5/103** (2006.01)    **G09B 19/00** (2006.01)
**A63B 24/00** (2006.01)

(52) Cooperative Patent Classification (CPC):
**A61B 5/112; A61B 5/4023; A61B 5/4836;**
**G16H 20/30; G16H 40/63;** A61B 5/0002;
A61B 5/1036; A61B 5/1038; A61B 5/1118;
A61B 5/6807; A61B 2505/09

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Feetme**
**75019 Paris (FR)**

(72) Inventors:
• **LAPLANCHE, Aurore**
**75019 Paris (FR)**
• **FERRÉ, Sabine**
**75019 Paris (FR)**

(74) Representative: **Icosa**
**83 avenue Denfert-Rochereau**
**75014 Paris (FR)**

(54) ## REHABILITATION METHOD

(57) The present invention relates to a rehabilitation method using a wearable sensor, said sensor monitoring activity of a patient during predetermined training program, computing a compliance index and an improvement index and updating the training program accordingly.

**FIG. 1**

## Description

## FIELD OF INVENTION

The present disclosure relates to a rehabilitation method using a wearable sensor, which monitors activity of a patient during training sessions and between training sessions and provides real-time biofeedback during training sessions, in everyday life. Compliance and behavioral improvement of the patient are evaluated and allow for updating of training.

## BACKGROUND OF INVENTION

In various medical conditions such as brain damage (stroke), nerve damage caused by accident (spinal cord injury) or degenerative diseases (brain damages) - in particular Parkinson's disease or Multiple Sclerosis - patients often temporary or durably loose the use of their limbs. For example, lower limbs damage leads to mobility impairments affecting quality of life such as difficulty to walk, balance instability or posture troubles. Following such injury, a period of rehabilitation is required during which gait and balance is recovered. Regular training may be also efficient to slow down effects of degenerative diseases.

In addition, difficulty with functional movements, e.g., an individual's gait, can lead to physical deconditioning and less activity, which contributes to poor cardiovascular fitness, muscular atrophy, and metabolic syndrome. These effects, in turn, can increase the risk of a stroke or cardiovascular event.

Such rehabilitation has been traditionally provided by a physiotherapist manually interacting with the patient. For example, the patient may walk on a treadmill or along a set path during which activity the physiotherapist will provide the desired instructions and feedback. These methods require continuous presence of a health care professional. Alternatively, robots or training machine are well known and used. Usually, these methods are run in a medical environment, where the patient needs to go: this represents an additional hurdle for the patient to comply with his/her rehabilitation work, especially for chronic diseases that require long-term rehabilitation care. Last, with these methods, the results of rehabilitation on everyday life of patient are not clearly measured, except by subjective assessment of patient and training program may be unsuitable. It is then difficult for physiotherapy's prescribers to assess efficacy of their prescription and to determine the need to pursue such care.

In view of the foregoing, there is a need for effective methods and systems for rehabilitation at home, in particular for gait and/or balance rehabilitation. An object of this disclosure aims at providing an improved rehabilitation method, in which a wearable sensor monitors the quality and the quantity of the training followed by a patient during a training program and monitors his/her activity in everyday life, allowing to adapt the training program upon compliance of the patient to said program on one hand and improvement of his/her everyday life activity on the other hand.

## SUMMARY

The present invention relates to a rehabilitation method comprising the following steps:

a) Providing to a patient a wearable sensor;
b) Defining for said patient a training program comprising a plurality of training sessions;
c) Acquiring exercise signal during some or all training sessions with the wearable sensor;
d) Acquiring everyday signal outside training sessions with the same wearable sensor;
e) Computing a compliance index from exercise signal;
f) Computing an improvement index from everyday signal; and
g) Updating the training program when compliance index is lower than a predetermined compliance threshold and/or improvement index is lower than a predetermined improvement threshold.

In an embodiment, an initial impairment of said patient is evaluated with the wearable sensor and the training program is defined according to said initial impairment.

In an embodiment, the wearable sensor comprises at least one sensor selected from a pressure sensor, a motion sensor, a posture sensor or a balance sensor.

In an embodiment, the wearable sensor is fitted in a footwear, preferably the wearable sensor is a pressure sensitive insole.

In an embodiment, updating the training program - step g) - comprises at least one of updating the number of sessions, updating the characteristics of one session, adding new sessions, deleting sessions, updating the settings of one exercise.

In other advantageous aspects of the invention, the training session is updated according to the following features, taken alone or in any possible combination:

- during a training session;
- after completion of a predetermined number of training sessions;
- automatically by a digital application;
- remotely.

In an embodiment, the wearable sensor provides the patient with a real-time feedback during a training session or with a feedback outside the training sessions in relationship with everyday activities

In an embodiment, the rehabilitation method targets gait rehabilitation and/or balance rehabilitation, in particular with the wearable sensor fitted in a footwear, preferably the wearable sensor is a pressure sensitive insole.

In an embodiment, the compliance index - computed in step e) - is a function of at least one of: the number of training sessions completed, the characteristics of training sessions completed, the settings of exercises completed, the level of completion of training sessions, the performance scores of the exercises of the session.

In an embodiment, the improvement index - computed in step f) - is a function of at least one of: global walking activity time monitored daily; spatio-temporal parameters of the walking activities such as total distance, maximum distance travelled without stopping, steps number, average speed, average stride length, number of stairs climbed or descended, phases of the step and plantar pressure parameters such as center of pressure trajectories, load, pressure distribution.

## DEFINITIONS

In the present invention, the following terms have the following meanings:

**"Activity":** refers to all movements performed by a patient and potentially measured by a wearable sensor. Activity refers to both quantity of the movements done (for example strides number, total distance travelled related to endurance, daily walking time, carried loads, limb flexion/extension/twist, standing-up or sitting down...) and quality of such movements (for example stride length, gait velocity, cadence, plantar pressure distribution, speed, angle of flexion...). In gait and/or balance rehabilitation methods, monitoring the following movements is especially relevant: walking, muscular reinforcement, posture balance and endurance.

**"Balance rehabilitation"** refers to exercises that strengthen the muscles that help keep the body upright, including legs and core. These exercises can improve stability and help prevent falls.

**"Gait rehabilitation"** refers to a wide range of physical therapy interventions, all aimed at improving the functional activity of ambulation and optimizing walking performance.

**"Rehabilitation"** refers to "a set of interventions designed to optimize functioning and reduce disability in individuals with health conditions in interaction with their environment" according to the World Health Organization.

## DETAILED DESCRIPTION

This invention relates to a rehabilitation method. In this method, a wearable sensor is provided to a patient, for instance suffering from mobility troubles. This sensor is wearable, so that the patient can keep the sensor all day long, without embarrassment, in his/her everyday activities. The sensor may be garment like a t-shirt, sockets, gloves or any strap fitted to the chest, hip, knee, ankle, shoulder or elbow. The wearable sensor may be also fitted in a footwear, for instance, the wearable sensor may be an insole.

The wearable sensor may be a motion sensor, like accelerometers or inertial motion units (IMU); a posture sensor; a balance sensor; a pressure sensor or any other sensor suitable to measure spatio-temporal parameters of a patient. In the specific case of gait and balance rehabilitation, it is especially relevant to measure gait parameters - stride length, step length, stride velocity, cadence, distance walked, strides number, gait cycle phases: stance and swing, stride duration, load - and plantar pressure distribution, center of pressure trajectory. Therefore, wearable sensors in the form of pressure sensitive insoles are advantageous. Pressure sensitive insoles may comprise a plurality of capacitive pressure cells. Capacitive pressure cells may comprise a thin sheet of deformable dielectric material placed between two electrodes. Indeed, the value of the capacitance C of a capacitive pressure cell can be determined as a function of the thickness L of the dielectric sheet of the capacitive pressure cells, the surface S of the upper electrode and the lower electrode of the capacitive pressure cells and the dielectric constant ε of the material between the electrodes by the following equation: $C = \frac{\varepsilon S}{L}$. Consequently, when the thickness L of the dielectric sheet of the capacitive pressure cells is changed, the capacitance C varies. Change of thickness may be induced by the pressure applied by the patient on the insole while walking or exercising. Such an insole enables measurement of the plantar pressure distribution versus time, leading to the dynamic parameters of gait.

The wearable sensor may be associated to a clock, so as to establish the exact date of signal acquisition, enabling to know when and how long the patient is exercising or is having everyday activities.

For said patient, a training program is defined. This program may be adapted to the patient to take into account his/her age, physical condition and disability. A training program typically lasts several weeks and comprises a determined number of sessions every week. Usually, at least three sessions every week are defined. Each session comprises a list of exercises, the latter being separated by resting time. The duration of the session - active time (number of exercises, number of repetitions for each exercise) and resting time (between each exercise) - is defined within the training program. These features are designated herein as session characteristics.

An exercise may be of any type used in rehabilitation methods. The duration and/or the number of repetitions and/or difficulty level - for instance a weight to be lifted or a stride length to achieve - of each exercise is defined within the training program, as well as the duration of resting time between repetitions in an exercise. An exercise may be also associated with a personalized objective. These parameters are designated herein as exercise settings. In the case of gait and/or balance, exercises may be selected from free-walking, balance working, flexibility exercises, controlled gait, muscular reinforcement such as plantar flexion, posture, and aerobic exercises.

An exercise may be static or dynamic, leading to motion, posture, pressure or balance signal acquired by the wearable sensor. In the case of gait and/or balance, the following features may be measured: spatio-temporal parameters such as cadence, stride length, gait velocity, distance, weight balance, center of pressure displacement, kinetic parameters with load and plantar pressure distribution including phases of the step such as heel strike and toe off.

Thanks to the wearable sensor, an exercise signal is acquired during some or all training sessions. Signal acquisition may start with an action of the patient - pushing a button on the sensor or launching an application controlling the wearable sensor - or may start autonomously when specific parameters corresponding to a session are identified by the wearable sensor. From exercise signal, it is possible to access to the compliance of the patient to his/her training program.

Outside the training sessions, the same wearable sensor acquires an everyday signal. This signal is an indication of patient activity in everyday life. By activity, it is meant here all movements performed by the patient and measured by the wearable sensor such as walking, standing, sitting, and resting. In particular, indication of patient activity may be a qualitative information: the patient did climb steps during the day; or a quantitative information: the patient did climb 20 steps in 1 minute three times in the day, the patient did walk with a speed of 1 meter per second, the gait was asymmetrical with a larger weight applied on right foot... In the case of gait and/or balance, the following features may be measured in everyday conditions: activity volume over a day (how active is the patient) with walking/sitting/standing and resting repartition, number of steps, total distance travelled, exact hours of such activities. Specifically, in the case of gait activities, all spatio-temporal parameters and plantar pressure distribution may be measured in everyday conditions.

In this method, the use of the same wearable sensor for acquisition of exercise signal and everyday signal is especially interesting for several reasons. The wearable sensor is not associated to training sessions only, and patient activity is monitored continuously without any additional burden. In addition, the same wearable sensor is used, so that signal acquired during training sessions may be compared with everyday signal. Finally, the rehabilitation method is adapted in real time - during the training program - according to compliance of the patient to the training program as well as according to a real-time evaluation of efficacy results of the training program, said efficacy being evaluated with spatio-temporal parameters from continuous monitoring of everyday life of the patient.

Then, a compliance index is computed from exercise signal. The compliance index may encompass various aspects.

First, the compliance index shows if the patient did practice the planned exercises. If it appears that exercises were not done, according to the wearable sensor, the patient may be called by the health care practician or by the coach to identify if there is a technical problem with the wearable sensor itself - hardware or software - or a lack of understanding regarding the training program or a lack of motivation from the patient or any specific situation that prevents the patient to practice.

Second, the compliance index shows how the patient did practice the exercises. If it appears that a specific exercise is not done in each session, the following list of remediation actions may be started: re-explaining exercise instructions; if no changes are measured after a predetermined number of sessions, update the exercise settings; if no changes are measured after a predetermined number of sessions, change the exercise, which is a way to update the training program. The same approach may be used if an exercise is completed, but with a low performance score. If it appears that patient assiduity is lowering globally on the whole training program, the patient may be called by the health care practician or by the coach to identify a lack of understanding regarding the training program or a lack of motivation from the patient.

Various parameters derived from exercise signal may be used to compute compliance index, such as number of training sessions completed and/or succeeded - i.e., completed successfully - the characteristics of training sessions completed and/or succeeded, the settings of exercises completed and/or succeeded, the level of completion and/or success - i.e., the efficacy result - of the training sessions, the performance scores of the exercises of the session.

In gait and/or balance rehabilitation, most relevant parameters to compute compliance index are: total duration of the program, number of training sessions/week completed and/or succeeded with for each session, number of exercises completed and/or succeeded, duration of the exercises completed and/or succeeded, number of repetitions/exercise completed and/or succeeded, duration of the repetitions/exercise completed and/or succeeded, difficulty levels and performance score of each exercise. Particularly useful compliance indices may be a parameter or a set of parameters or a function of some parameters - typically a ratio of parameters.

Then, an improvement index is computed from everyday signal. The improvement index is based on successive evaluations showing that an activity is improving or degrading and/or the quality of an activity is improving or degrading. Various parameters derived from everyday signal may be used to compute improvement index, such as activity parameters: walking/sitting/standing and resting, spatio-temporal parameters such as distance, walking speed and plantar pressure distribution, load. For instance, the total distance travelled by the patient every day may be increasing, showing that the patient is more mobile. To the contrary, the length of resting times during day may be increasing, showing that the patient is not moving at all. Or the measure of gait shows that asymmetry is unchanged, showing that exercises are not efficient to improve the patient's state. Suitable improvement index may be one of a global walking activity time monitored daily or a spatio-temporal parameter of the walking activity such as total distance, maximum distance travelled without stopping, steps number, average speed, average stride length, number of stairs climbed or descended, phases of the step and plantar pressure parameters such as center of pressure trajectories, load, pressure distribution.

In gait and/or balance rehabilitation, most relevant parameters to compute improvement index are linked to gait and/or linked to general activity. Regarding gait, cadence, distance, stride length, walking speed, gait cycle phases and plantar pressure distribution are relevant parameters. Regarding general activity, relevant parameters may be selected from: the total distance travelled by the patient every day, the maximum distance travelled without stopping, the number of steps, the average speed while walking, average stride length, the number of stairs climbed or descended... Particularly useful improvement indices may be a parameter or a set of parameters or a function of some parameters - typically a ratio of parameters.

Finally, when compliance index is lower than a predetermined threshold, and/or when improvement index is lower than a predetermined threshold, the training program is updated. The update may address any aspect of the training program. In particular, the number and/or duration of training sessions may be updated - addition or deletion of a session for instance. Besides, the sessions characteristics and /or the exercises settings may be updated.

The training program may be updated in several ways. First, in real-time, during exercises, compliance index and/or improvement index may be computed and lead to an update of the current training session, for instance by increasing resting time between exercises. Updating in real-time is advantageous as it limits the risk of exercising in a hazardous way regarding the state of the patient. Alternatively, after completion of a predetermined number of training sessions, an update of training program may be done based on compliance index and/or improvement index. This update *a-posteriori* is advantageous as it encompasses all achievements over a long period and lowers the variations of performance or compliance of the patient linked to other constraints in everyday life.

In an embodiment, compliance index and improvement index are monitored to establish trends, such as patient disinterest or inefficacy of an exercise, then send alerts either to the patient or to the health care practitioner.

In an embodiment, the wearable sensor is used in a preliminary step to evaluate impairment or disabilities of the patient. The initial impairment may be used to define the training program of the patient in a personalized manner. In addition, the initial impairment may be used as a reference state for improvement index computation.

In an embodiment, the wearable sensor provides the patient with a feedback. By feedback, it is meant herein that an information is sent or displayed to the patient. Accordingly, the wearable sensor may be connected to a communication network so as to send information to an external party or may comprise a display.

In particular, a feedback may be provided in real-time during a training session, based on an information derived from exercise signal. The feedback may be elaborated during an exercise, to alert the patient that instructions for said exercise are not correctly followed and/or to guide the patient. The feedback may be elaborated during a resting time between two exercises. This real-time feedback is an alternative to the presence of a physiotherapist assisting the patient. It enables a correct completion of exercises at home, without health care professional supervision.

In another embodiment, the wearable sensor provides the patient with a feedback outside the training sessions, in relationship with everyday activities. Such feedback is especially valuable as it allows to note improvements in everyday life, which is satisfying for the patient.

In gait and/or balance rehabilitation, real-time feedback may be an information or display of distance, gait velocity, cadence, stride length, center of pressure trajectories, body weight displacement, asymmetry or load.

In an embodiment, the training program is supported by a digital application. The digital application may be run on a device comprising computing means such as a smartphone, a tablet, a computer or a wearable device worn by the patient. Said computing means may be used to compute compliance index and improvement index.

The digital application may be connected to a communication network so as to send information to an external party, such as a health care specialist or a coach or a cloud computing system, and to receive information from an external party. Said information may be the exercise signal and everyday signal for review by a health care specialist or for computing of compliance index and improvement index. Said information may be a real-time feedback or an alert during an exercise not correctly completed. Said information may be the update of the training program or messages from a health care specialist or a coach, in particular technical advices and/or cheers when improvement is measured. This remote system is an alternative to the presence of a physiotherapist assisting the patient.

In an embodiment, the update of the training program may be automatic and provided by a digital application. Alternatively, the update of the training program is remote, i.e., defined by a health care practician having access to signals and indices remotely, and update is sent to patient by a digital application.

**BRIEF DESCRIPTION OF THE DRAWINGS**

**Figure 1** is a graph showing the daily record of real practice time during training sessions.

**Figure 2** is a graph showing the weekly record of practice time for an exercise.

**EXAMPLES**

The present invention is further illustrated by the following examples.

Example 1: Program update according to patient's functional capabilities.

The initial rehabilitation program for the patient includes 3 training sessions a week in addition to 30 minutes of free walking per week - an everyday activity. Each training session contains the following exercises: 5 minutes of balance, 5 minutes of gait quality, 5 minutes of muscular reinforcement and 15 minutes of walking (aerobic capacity). The effective working time per session is 30 minutes and a total of 2 hours/week (3 training sessions plus free walking) is expected to be practiced at home.

Pressure sensitive insoles are provided to the patient. The insoles are connected with Bluetooth Low Energy to a smartphone running a digital application. The insoles measure continuously motion of patient as well as plantar pressure and send these signals to the smartphone or store the signal in an internal memory if connection with smartphone is not available. When the patient starts a training session, he hits a starting button on the smartphone. During exercises, pressure sensitive insoles allow determination of real practice time: indeed, all exercises have a specific signature in terms of motion and plantar pressure, and resting times are well identified between exercises. Besides, walking time is easily monitored (no stops, regular and high speed, plantar pressure distribution...). After each session, a compliance index is computed, namely the ratio between the real practice time and the expected practice time. The same compliance index is computed for free walking time.

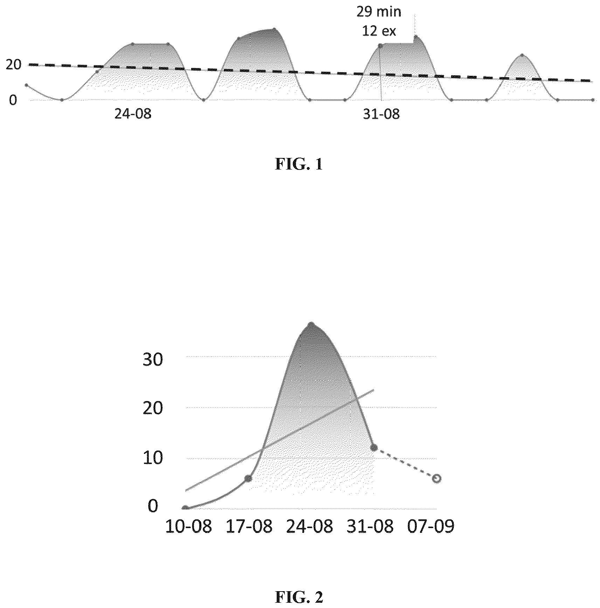

Figure 1 shows a daily record of real practice time during training sessions for two weeks: each point corresponds to one day. On August the 31st, patient did exercise 29 minutes and completed 12 exercises. Clearly, compliance of patient is decreasing: in the second week, sessions are not done every two days and average real practice time decreases (dotted line) to be finally shorter than 10 minutes, resulting in a compliance index lower than 50%.

Figure 2 shows the weekly record of walking (aerobic capacity) time (expected time of 3x15 minutes every week): each point corresponds to one week. Again, the patient is not practicing enough during the second week.

These data are sent every week to a coach in charge of the follow-up, she performed a compliance analysis on the online platform. The first week of use, the coach noticed that the patient completed only up to 50% of his/her program (1 hour of real practice duration instead of 2 hours expected). The patient has not practiced enough. After analyzing the detail of the practice duration per exercise for all the training sessions, the coach observed that the patient did not perform totally for each training session the same exercise (aerobic capacity exercise) and did not perform at all the 30 minutes of free walking, as revealed by the improvement index based on the measure of daily free walking time.

After gathering all these observations, a phone call was made to the patient. It turned out that the patient had some difficulties walking for a long time. Thus, all the aerobic exercises were not adapted to his/her functional capacities. This was confirmed by the compliance index. Therefore, the free walking was removed and the total duration of aerobic capacity exercise was decreased (from 15 minutes to 5 minutes per session). The following week, the patient reached 100% of program compliance. Simultaneously, the daily free walking time - measured in everyday conditions, not associated to a specific exercise - increased, and led to a positive improvement index: program update did help improving the patient conditions.

Example 2: Program update according to patient's difficulties.

The initial rehabilitation program for the patient includes 5 training sessions of 40 minutes per week in addition to a 30 minutes of free walking per week. The expected working time per week is 3 hours and 50 minutes. The patient uses the same pressure sensitive insoles as in example 1.

After 9 weeks of rehabilitation, the compliance index shows that the patient has a compliance of 100%: he is performing exercise without obvious difficulties. However, the compliance index of a specific exercise - balance and speed - computed by plantar pressure measurements acquired during exercise is too low: between 46 and 60%, below a threshold of 80% predetermined for this type of exercise.

Therefore, the training program is updated by replacing the balance and speed exercise by a posturofeedback exercise, which is less difficult. Later, when the patient will reach a compliance index of 80% on a regular basis for posturofeedback exercise, the training program will be updated again to practice the initial balance and speed exercise. In this case, the initial training program was not suitable and the change of exercise allowed the patient to practice an exercise suitable for his/her current state.

Example 3: Program adaptation according to the efficacy data measured in real-life.

Here the patient was in his fifth month of self-rehabilitation and was using the same pressure sensitive insoles as in example 1. Global compliance index was 96% and compliance index for each exercise in training sessions demonstrated a high percentage of success. Last, the results during the weekly 6 minutes walking tests were also showing a slight positive improvement in the gait quality with a higher velocity and longer stride length measured with pressure sensitive insoles during exercise. However, the global daily activities of the patient didn't increase over time. The improvement index computed with the maximum distance travelled without stopping was almost null: there were no distance travelled without stopping longer than 350 meters, as recorded with pressures sensitive insoles worn by the patient all day long. Based on this remote data available, a phone call with the patient helped to figure out that he didn't feel more independent or confident in his mobility because of his fatigability. Thus, the program was updated with endurance exercises and adjusted to decrease post exertional fatigue. A few weeks later, maximum distance travelled without stopping increased leading to an improvement of patient's conditions.

**Claims**

1. A rehabilitation method comprising:

   **a)** Providing to a patient a wearable sensor;
   **b)** Defining for said patient a training program comprising a plurality of training sessions;
   **c)** Acquiring exercise signal during some or all training sessions with the wearable sensor;
   **d)** Acquiring everyday signal outside training sessions with the same wearable sensor;
   **e)** Computing a compliance index from exercise signal;
   **f)** Computing an improvement index from everyday signal; and
   **g)** Updating the training program when compliance index is lower than a predetermined compliance threshold and/or improvement index is lower than a predetermined improvement threshold.

2. The rehabilitation method according to claim 1, wherein an initial impairment of said patient is evaluated with the wearable sensor and wherein the training program is defined according to said initial impairment.

3. The rehabilitation method according to claim 1 or 2, wherein the wearable sensor comprises at least one sensor selected from a pressure sensor, a motion sensor, a posture sensor or a balance sensor.

4. The rehabilitation method according to any one of claims 1 to 3, wherein the wearable sensor is fitted in a footwear, preferably the wearable sensor is a pressure sensitive insole.

5. The rehabilitation method according to any one of claims 1 to 4, wherein updating the training program comprises at least one of updating the number of sessions, updating the characteristics of one session, adding new sessions, deleting sessions, updating the settings of one exercise.

6. The rehabilitation method according to any one of claims 1 to 5, wherein the training program is updated during a training session.

7. The rehabilitation method according to any one of claims 1 to 6, wherein the training program is updated after completion of a predetermined number of training sessions.

8. The rehabilitation method according to any one of claims 1 to 7, wherein the wearable sensor provides the patient with a real-time feedback during a training session or with a feedback outside the training sessions in relationship with everyday activities.

9. The rehabilitation method according to any one of claims 1 to 8, wherein rehabilitation method targets gait rehabilitation and/or balance rehabilitation.

10. The rehabilitation method according to claim 9, wherein the wearable sensor is fitted in a footwear, preferably the wearable sensor is a pressure sensitive insole.

11. The rehabilitation method according to any one of claims 1 to 10, wherein the compliance index is a function of at least one of: the number of training sessions completed, the characteristics of training sessions completed, the settings of exercises completed, the level of completion of training sessions, the performance scores of the exercises of the session.

12. The rehabilitation method according to any one of claims 1 to 11, wherein the improvement index is a function of at least one of: global walking activity time monitored daily; spatio-temporal parameters of the walking activities such as total distance, maximum distance travelled without stopping, steps number, average speed, average stride length, number of stairs climbed or descended, phases of the step and plantar pressure parameters such as center of pressure trajectories, load, pressure distribution.

13. The rehabilitation method according to any one of claims **1** to **12,** wherein updating the training program is automatic and provided by a digital application.

14. The rehabilitation method according to any one of claims **1** to **13,** wherein updating the training program is remote.

**FIG. 1**

**FIG. 2**

Europäisches
Patentamt

European
Patent Office

Office européen
des brevets

# EUROPEAN SEARCH REPORT

Application Number

EP 21 30 5716

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/236867 A1 (AVNI ARIK [IL] ET AL) 12 September 2013 (2013-09-12) * paragraphs [0247], [0360] - [0361], [0383] - [0390]; figure 77 * | 1-14 | INV. A61B5/103 ADD. G09B19/00 A63B24/00 |
| X | US 2003/027118 A1 (ABRAHAM-FUCHS KLAUS [DE] ET AL) 6 February 2003 (2003-02-06) * the whole document * | 1 | |
| X | US 2004/059543 A1 (ABRAHAM-FUCHS KLAUS [DE] ET AL) 25 March 2004 (2004-03-25) * paragraph [0023]; figures 1,2 * | 1 | |
| X | US 2020/054931 A1 (MARTIN KEVIN L [US] ET AL) 20 February 2020 (2020-02-20) * paragraphs [0141] - [0142] * | 1 | |
| X | US 2014/172442 A1 (BRODERICK JEFF [US] ET AL) 19 June 2014 (2014-06-19) * the whole document * | 1 | |

TECHNICAL FIELDS
SEARCHED       (IPC)

A61B
G09D
G09B
A63B
G16H

The present search report has been drawn up for all claims

| Place of search | Date of completion of the search | Examiner |
|---|---|---|
| Berlin | 10 November 2021 | Kronberger, Raphael |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

......................................................................
& : member of the same patent family, corresponding
    document

EPO FORM 1503 03.82 (P04C01)

EP 4 094 684 A1

## ANNEX TO THE EUROPEAN SEARCH REPORT
## ON EUROPEAN PATENT APPLICATION NO.

EP 21 30 5716

10-11-2021

| Patent document cited in search report | | Publication date | Patent family member(s) | | Publication date |
|---|---|---|---|---|---|
| US 2013236867 | A1 | 12-09-2013 | US | 2013236867 A1 | 12-09-2013 |
| | | | WO | 2013134330 A1 | 12-09-2013 |
| US 2003027118 | A1 | 06-02-2003 | CA | 2395216 A1 | 27-01-2003 |
| | | | EP | 1279416 A2 | 29-01-2003 |
| | | | JP | 2003132147 A | 09-05-2003 |
| | | | US | 2003027118 A1 | 06-02-2003 |
| US 2004059543 | A1 | 25-03-2004 | EP | 1378849 A1 | 07-01-2004 |
| | | | US | 2004059543 A1 | 25-03-2004 |
| US 2020054931 | A1 | 20-02-2020 | US | 2020054931 A1 | 20-02-2020 |
| | | | WO | 2019232455 A1 | 05-12-2019 |
| US 2014172442 | A1 | 19-06-2014 | NONE | | |

EPO FORM P0459

For more details about this annex : see Official Journal of the European Patent Office, No. 12/82